# EUROPEAN PATENT SPECIFICATION

(11) **EP 3 989 159 B1**
(45) Date of publication and mention of the grant of the patent: **17.12.2025**
(21) Application number: 20800702.1
(22) Date of filing: 08.09.2020
(51) Int. Cl.: G06T 7/00, A61B 5/103, A61B 5/11

(54) **SYSTEM FOR THE BIOMECHANICAL STUDY OF THE MOVEMENT OF A PERSON**
SYSTEM ZUR BIOMECHANISCHEN UNTERSUCHUNG DER BEWEGUNG EINER PERSON
SYSTÈME POUR L'ÉTUDE BIOMÉCANIQUE DU MOUVEMENT D'UN INDIVIDU

(30) Priority: 22.08.2020 ES 202000416 U
(43) Date of publication of application: 27.04.2022
(73) Proprietor: SÁNCHEZ-OSORIO NICOLÁS, Eduardo, 08960 Sant Just Desvern, Barcelona (ES)
(72) Inventor: SÁNCHEZ-OSORIO NICOLÁS, Eduardo, 08960 Sant Just Desvern, Barcelona (ES)
(74) Representative: Gallego Jiménez, José Fernando
(86) International application number: PCT/ES2020/000041
(87) International publication number: WO 2022/043590

(56) References cited:
- EP-A1- 2 308 373
- KR-B1- 100 969 038
- US-A1- 2017 068 774

## Description

### Technique sector

This invention refers to a system that allows to assess the biomechanics of an individual's motion while walking, running or performing specific sports gestures. Said system has a computer system fitted with intelligent software that measures and provides real-time parameterisation of the individual's dynamic bodily behaviour and the pressure exerted by said individual's feet on the treading surface while performing an exercise, it automatically prepares a diagnosis and a treatment based on customised insoles generated from the data obtained during the assessment performed. This system is applicable in the fields of biomechanics, podiatry, sports medicine, physiotherapy, readjustment and physical preparation.

### Status of the previous technique

At present, the biomechanical studies consisting of the analysis of the way of stepping are widely known. These studies are carried out by observing the different treading and pressure areas of feet on a surface.

When some kind of anomaly is detect in the footfall, the usual solution is the use of corrective insoles, however the current technique does not thoroughly analyse the movements of the entire body while performing exercise. At best, a simple observation is made of the individual while performing an exercise, which prevents the analytical determination of the body's movements that lead to a poor footfall and, therefore, not allowing to correct the cause of said poor footfall.

Other techniques involving the observation of an individual's movements during exercise require the use of specific markers and the use of a calibrated space to determine the individual's position. These techniques do not allow for the automatic preparation of a biomechanical diagnosis and for relating this to the injuries that the individual is suffering or may suffer, in order to prevent them.

The applicant of the invention does not know of the existence in present day podiatry of any dynamic assessment system capable of assessing and measuring the kinetic and kinematics of the human body in real-time. So far everything has been based on the experience and power of observation of the specialist's eye, but no numerical results are obtained to validate said observation.

Prior art on biomechanical studies for custom footwear design is disclosed in EP2308373A1 and US2017/068774A1.

### Explanation of the invention

The system for the biomechanical study of an individual's motion, purpose of this invention, allows to perform a three-dimensional study of an individual's entire body in real-time, and to analyse the kinetics and kinematics of the individual for maintaining balance in bipedal and when walking and running. This system also allows to analyse the biomechanics of specific sports gestures found in different sports, such as football, basketball, tennis, paddle tennis, cycling, etc.

This system is based on the combination of the concept of three-dimensional vision and continuous monitoring of foot plantar pressure in real-time to be able to monitor and parameterise body motion. All this is integrated into a system capable of working autonomously, following working protocols validated by ergodynamics.

The system has its own intelligence to be able to automatically diagnose different pathologies in the process of motion of the human body by studying the kinematic and dynamic motion that intervenes in the process of walking and/or running, comparing the data obtained with an extensive database that is the result of long-standing experience. This system is unlike other technologies that only focus on the study of the lower limbs (feet) of the human body.

According to the invention, this system comprises the features as defined in appended claim 1.

According to the invention, the real-time video capture cameras can comprise one or more RGB-D vision sensors capable of measuring the depth at which the objects in the image are, or multiple fixed and calibrated sensors.

The video capture cameras used are suitable and capable of delivering 60 to 90 fps, that is, two or three times what the human eye is capable of seeing. This allows to view movements focused on detecting abnormalities in the individual's motion.

Image processing allows to process and analyse in real-time the initial position (static) of the individual and its kinematic and dynamic evolution during exercise and have real-time access to the evolution of the joint angles, angular velocity and position in the three planes of space of the more relevant anatomical references such as the knees, hips and spine.

The software responsible for processing the images provided by the vision sensors comprises an algorithm that records 3D point clouds in an single point cloud and an optimisation algorithm that extracts a representation of the individual's skeleton from each image.

The processing of such images eventually includes the application of a smoothing technique, the post-processing of the sequence of skeletons in order to eliminate estimation noise from the independent frames and the processing to obtain the data concerning the movements: positions, angles, linear or angular velocities and linear or angular accelerations in the three planes of space.

The system allows users to configure each metric independently, from the specialist user interface, by setting the normal/poor/optimal/pathological ranges from a the point of view of interest or clinical use. These ranges are used to detect points of interest in the sequence of images in which the individual performs movements that are outside what is considered as the normal range.

The system is configured to allow two modes of operation:
- a streaming/recording mode, in which data is sent to the user interface, at the same time as being recorded on a hard drive within the system, and
- a playback mode, in which the data saved in a user interface is loaded for scanning.

The system also comprises a user interface for the individual, which plays the video and shows the posture, the pressure image of the feet, and a series of markers with the angles determined by the specialist.

The processing of baropodometric images involves the application of a local optimisation algorithm at subpixel level to temporarily align the sequence of such baropodometric images, determining the displacement between consecutive frames. This information is used to extract the historical image of the footsteps, displaying the entire temporal sequence in space.

Capturing baropodometric images allows to obtain data that would otherwise be impossible to gather using a small static platform, and to correlate the detrimental mechanism, at any level, with the behaviour of the foot and of the individual's balance at each moment while exercising.

In addition to providing all analytical data for the individualised biomechanical study and adjusted to the individual's age and activity, the system is able to identify biomechanical alterations that are the cause of an injury, or that may pose a risk of injury to the individual.

The system itself is designed so that the obtained data can lead to a biomechanical diagnosis and designing the best fully customised treatment (insoles) for the alterations.

One of the features of the system is that it combines three dimensions: length, width and depth of 3D technology, with the dimension of time, therefore data collection takes place in real time and space, allowing users to access all the information instantly and to immediately determine the action plan to be implemented.

### Brief description of the drawings' content.

To complement the description provided and for the purpose of helping to understand the features of the invention, this description is accompanied by a set of drawings for illustrative but not limiting purposes, representing the following:

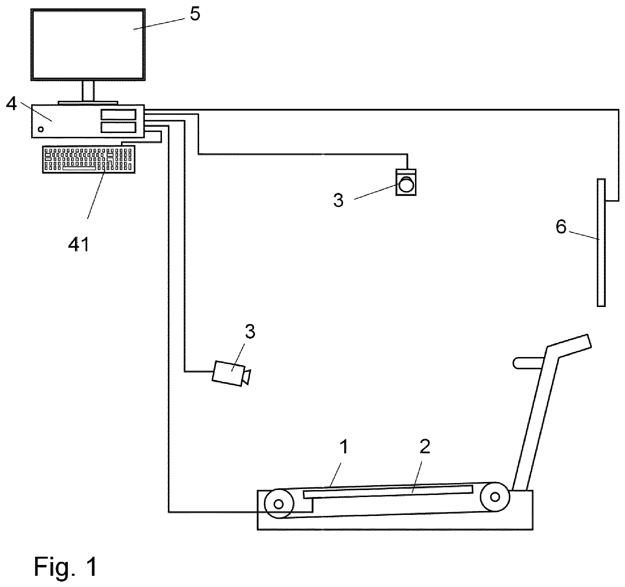

- Figure 1 shows a diagram of the system for the biomechanical study of an individual's motion, according to the intervention.

### Detailed display of mode for implementing the invention.

In the example of implementation shown in figure 1, the components of the invention system have been represented schematically, comprising: - a treadmill (1) fitted with a pressure platform (2) equipped with a high-resolution pressure sensor, which obtains real-time baropodometric images of the individual's footfall as he/she walks or runs on said treadmill; - real-time video capture media (3) of the individual's body posture while exercising on the treadmill and, - a computer system (4) equipped with intelligent software, to which the video capture media (2) and said pressure platform (3) are connected.

The video capture media (3) are in this case cameras with fixed and calibrated sensors, and are suitable for delivering between 60 and 90 fps (frames per second).

The system comprises a user interface (41) for a specialist responsible for controlling the execution mode of the computer software and the information of the individual that is displayed on a screen (5) and a user interface (6) for the individual, represented by a screen showing the individual's video and posture during exercise, the image of the foot pressures, and a series of markers with different angles of movement.

The intelligent software of the computer equipment processes the information provided by the video capture media and the baropodometric images provided by the pressure platform, provides analytical data for the individual's personalised biomechanical study, identifying the biomechanical alterations that are the cause of an injury, or which may pose a risk of injury to the individual, and eventually designs a fully personalised treatment using insoles which are designed to correct the detected alterations.

Once the nature of the invention has been sufficiently described, as well as an example of preferential implementation, it is stated for the appropriate purposes that the materials, shape, size and arrangement of the described elements may be modified, provided this does not alter the essential features of the invention as claimed below.

## Claims

1. System for the biomechanical study of an individual's motion; **characterised by** comprising:
- a treadmill (1) fitted with a pressure platform (2) equipped with a high-resolution pressure sensor configured to obtain information in the form of real-time high-resolution baropodometric images of the individual's footfall while walking or running on the treadmill;
- real-time video capture media (3) configured to obtain information of the individual's body posture while exercising on the treadmill;
- computer equipment (4) to which the following are connected: the real-time video capture media (3) and the pressure platform (2), a user interface (41) for a specialist in charge of controlling the execution mode of the software and the individual's information that is displayed on a screen (5), wherein the computer equipment is equipped with intelligent software that includes algorithms that are configured for:
- processing the information obtained by the real-time video capture media (3) to obtain data related to positions, angles, speeds and accelerations of the individual's movements in the three planes of space, and comparing them with a range of predetermined values, considered normal, deficient, optimal and pathological, such that those movements of the individual that are outside normal values are determined;
- processing the information obtained in the form of baropodometric images by the pressure platform (2), dynamically analysing the distribution of the pressures over time and including the entire sequence of footfalls extracted, rejecting the footfalls that do not follow the normal pattern of the sequence, and obtaining an average pressure distribution model;
- providing analytical data for the individual's personalised biomechanical study, by comparing the information obtained by the real-time video capture media (3) and by the pressure platform (2) with a database, so that biomechanical alterations that are the cause of an injury, or which may pose a risk of injury to the individual, are automatically identified; and
- designing a fully personalised treatment using insoles which are designed to correct the identified biomechanical alterations.

2. System, according to claim 1, **whereby** the real-time video capture media (3) comprise one or more RGB-D vision sensors capable of measuring the depth at which the objects in the image are located.

3. System, according to claim 1, **whereby** the real-time video capture media (3) comprise one or more fixed and calibrated sensors.

4. System, according to any of the previous claims, **whereby** the real-time video capture media (3) are suitable for delivering between 60 and 90 fps.

5. System, according to any of the previous claims, **whereby** the system comprises a user interface (6) for the individual, which shows the video and posture, the image of pressures of the feet, and a series of markers with different angles of movement.

6. System, according to claim 1, **whereby** the intelligent software for processing the images provided by the real-time video capture media (3) comprises an algorithm configured to register 3D point clouds in a single point cloud and an optimisation algorithm configured to extract for each image a representation of the individual's skeleton, and wherein the intelligent software is further configured to apply a smoothing technique, to post-process a sequence of representations of the individual's skeletons and to obtain the data concerning the movements: positions, angles, linear or angular velocities and linear or angular accelerations in the three planes of space.

7. System, according to claim 1, **whereby** the intelligent software comprises a local optimisation algorithm at subpixel level that temporarily aligns the sequence of such baropodometric images, determines the displacement between consecutive frames; and extracts the historical image of the footfalls, displaying the entire temporal sequence in space.

## Patentansprüche

1. System für die biomechanische Untersuchung der Bewegung einer Person; **dadurch gekennzeichnet, dass** es Folgendes umfasst:
- ein Laufband (1), das mit einer Druckplattform (2) ausgestattet ist, die mit einem hochauflösenden Drucksensor versehen ist, der dazu konfiguriert ist, Informationen in Form von hochauflösenden baropodometrischen Bildern in Echtzeit über den Fußabdruck der Person zu erhalten, während diese auf dem Laufband geht oder läuft;
- Echtzeit-Videoerfassungsmedien (3), die dazu konfiguriert sind, Informationen über die Körperhaltung der Person während des Trainings auf dem Laufband zu erhalten;
- Rechnereinrichtung (4), an die Folgendes angeschlossen ist: die Echtzeit-Videoerfassungsmedien (3) und die Druckplattform (2), eine Benutzerschnittstelle (41) für eine Fachkraft, die für das Steuern des Ausführungsmodus der Software und der Informationen zur Person zuständig ist, die auf einem Bildschirm (5) angezeigt werden, wobei die Rechnereinrichtung mit einer intelligenten Software versehen ist, die Algorithmen enthält, die zu Folgendem konfiguriert sind:
- Verarbeiten der Informationen, die durch die Echtzeit-Videoerfassungsmedien (3) erhalten wurden, um Daten zu erhalten, die sich auf Positionen, Winkel, Geschwindigkeiten und Beschleunigungen der Bewegungen der Person in den drei Raumebenen beziehen, und Vergleichen dieser mit einem Bereich vorbestimmter Werte, die als normal, mangelhaft, optimal und pathologisch angesehen werden, sodass diejenigen Bewegungen der Person, die außerhalb der normalen Werte liegen, bestimmt werden;
- Verarbeiten der Informationen, die in Form von baropodometrischen Bildern durch die Druckplattform (2) erhalten werden, dynamisches Analysieren der Verteilung der Drücke im Laufe der Zeit und beinhaltend die gesamte Sequenz der extrahierten Fußabdrücke, Zurückweisen der Fußabdrücke, die nicht dem normalen Muster der Sequenz folgen, und Erhalten eines durchschnittlichen Druckverteilungsmodells;
- Bereitstellen von Analysedaten für die personalisierte biomechanische Untersuchung der Person, indem die Informationen, die durch die Echtzeit-Videoerfassungsmedien (3) und durch die Druckplattform (2) erhalten werden, mit einer Datenbank verglichen werden,
sodass biomechanische Veränderungen, die die Ursache für eine Verletzung sind oder ein Verletzungsrisiko für die Person darstellen können, automatisch identifiziert werden; und
- Gestalten einer vollständig personalisierten Behandlung unter Verwendung von Einlegesohlen, die dazu gestaltet sind, die identifizierten biomechanischen Veränderungen zu korrigieren.

2. System nach Anspruch 1, **wobei** die Echtzeit-Videoerfassungsmedien (3) einen oder mehrere RGB-D-Vision-Sensoren umfassen, die in der Lage sind, die Tiefe zu messen, in der sich die Objekte auf dem Bild befinden.

3. System nach Anspruch 1, **wobei** die Echtzeit-Videoerfassungsmedien (3) einen oder mehrere fixierte und kalibrierte Sensoren umfassen.

4. System nach einem der vorhergehenden Ansprüche, **wobei** die Echtzeit-Videoerfassungsmedien (3) geeignet sind, zwischen 60 und 90 fps zu liefern.

5. System nach einem der vorhergehenden Ansprüche, **wobei** das System eine Benutzerschnittstelle (6) für die Person umfasst, die das Video und die Körperhaltung, das Bild der Drücke der Füße und eine Reihe von Markierungen mit verschiedenen Bewegungswinkeln zeigt.

6. System nach Anspruch 1, **wobei** die intelligente Software zum Verarbeiten der Bilder, die durch die Echtzeit-Videoerfassungsmedien (3) bereitgestellt werden, einen Algorithmus, der dazu konfiguriert ist, 3D-Punktwolken in einer einzigen Punktwolke zu registrieren, und einen Optimierungsalgorithmus umfasst, der dazu konfiguriert ist, für jedes Bild eine Darstellung des Skeletts der Person zu extrahieren, und wobei die intelligente Software ferner dazu konfiguriert ist, eine Glättungstechnik anzuwenden, eine Sequenz von Darstellungen der Skelette der Person nachzubearbeiten und die Daten bezüglich der Bewegungen zu erlangen: Positionen, Winkel, Linear- oder Winkelgeschwindigkeiten und Linear- oder Winkelbeschleunigungen in den drei Raumebenen.

7. System nach Anspruch 1, **wobei** die intelligente Software einen lokalen Optimierungsalgorithmus auf Subpixel-Ebene umfasst, der die Sequenz solcher baropodometrischen Bilder vorübergehend ausrichtet, die Verschiebung zwischen aufeinanderfolgenden Frames bestimmt; und das historische Bild der Fußabdrücke extrahiert, wobei die gesamte zeitliche Sequenz im Raum angezeigt wird.

## Revendications

1. Système pour l'étude biomécanique du mouvement d'un individu ; **caractérisé en ce qu'**il comprend :
- un tapis roulant (1) doté d'une plate-forme de pression (2) équipée d'un capteur de pression haute résolution configuré pour obtenir des informations sous la forme d'images baropodométriques haute résolution en temps réel des pas de l'individu pendant qu'il marche ou court sur le tapis roulant ;
- un support de capture vidéo en temps réel (3) configuré pour obtenir des informations sur la posture de corps de l'individu pendant qu'il fait de l'exercice sur le tapis roulant ;
- un équipement informatique (4) auquel les éléments suivants sont connectés : le support de capture vidéo en temps réel (3) et la plate-forme de pression (2), une interface utilisateur (41) pour un spécialiste chargé de contrôler le mode d'exécution du logiciel et les informations de l'individu qui sont affichées sur un écran (5), dans lequel l'équipement informatique est équipé de logiciel intelligent qui comporte des algorithmes qui sont configurés pour :
- traiter les informations obtenues par le moyen de capture vidéo en temps réel (3) pour obtenir des données relatives aux positions, aux angles, aux vitesses et aux accélérations des mouvements de l'individu dans les trois plans de l'espace, et les comparer à une plage de valeurs prédéterminées, considérées comme normales, déficientes, optimales et pathologiques, de telle sorte que ces mouvements de l'individu qui se situent en dehors des valeurs normales sont déterminées ;
- traiter les informations obtenues sous forme d'images baropodométriques par la plate-forme de pression (2), analysant dynamiquement la distribution des pressions au fil du temps et comportant la totalité de la séquence de pas extraite, rejetant les pas qui ne suivent pas le modèle normal de la séquence, et obtenant un modèle de distribution de pression moyenne ;
- fournir des données analytiques pour l'étude biomécanique personnalisée de l'individu, en comparant les informations obtenues par le support de capture vidéo en temps réel (3) et par la plate-forme de pression (2) avec une base de données,
de sorte que des altérations biomécaniques qui sont à l'origine d'une blessure, ou qui peuvent constituer un risque de blessure pour l'individu, sont automatiquement identifiées ; et
- concevoir un traitement entièrement personnalisé à l'aide de semelles conçues pour corriger les altérations biomécaniques identifiées.

2. Système, selon la revendication 1, **moyennant quoi** les supports de capture vidéo en temps réel (3) comprennent un ou plusieurs capteurs de vision RVB-D capables de mesurer la profondeur à laquelle les objets dans l'image sont situés.

3. Système, selon la revendication 1, **moyennant quoi** les supports de capture vidéo en temps réel (3) comprennent un ou plusieurs capteurs fixes et calibrés.

4. Système, selon l'une quelconque des revendications précédentes, **moyennant quoi** les supports de capture vidéo en temps réel (3) sont adaptés pour délivrer entre 60 et 90 frames par seconde.

5. Système, selon l'une quelconque des revendications précédentes, **moyennant quoi** le système comprend une interface utilisateur (6) pour l'individu, qui présente la vidéo et la posture, l'image de pressions des pieds, et une série de marqueurs avec différents angles de mouvement.

6. Système, selon la revendication 1, **moyennant quoi** le logiciel intelligent pour le traitement des images fournies par le support de capture vidéo en temps réel (3) comprend un algorithme configuré pour enregistrer des nuages de points 3D dans un nuage de points unique et un algorithme d'optimisation configuré pour extraire pour chaque image une représentation du squelette de l'individu, et dans lequel le logiciel intelligent est en outre configuré pour appliquer une technique de lissage, pour post-traiter une séquence de représentations du squelette de l'individu et pour obtenir les données concernant les mouvements : les positions, les angles, les vitesses linéaires ou angulaires et les accélérations linéaires ou angulaires dans les trois plans de l'espace.

7. Système, selon la revendication 1, **moyennant quoi** le logiciel intelligent comprend un algorithme d'optimisation locale au niveau du sous-pixel qui aligne temporairement la séquence de telles images baropodométriques, détermine le déplacement entre des frames consécutives ; et extrait l'image historique des pas, affichant la totalité de la séquence temporelle dans l'espace.
